# EUROPÄISCHE PATENTSCHRIFT

(11) **EP 2 958 602 B1**
(45) Veröffentlichungstag und Bekanntmachung des Hinweises auf die Patenterteilung: **16.05.2018**
(21) Anmeldenummer: 14702595.1
(22) Anmeldetag: 04.02.2014
(51) Int. Cl.: A61L 26/00, A61K 9/00, A61K 9/06, A61K 47/12

(54) **ZUSAMMENSETZUNG ZUR BESCHLEUNIGTEN WUNDHEILUNG GESCHÄDIGTEN GEWEBES**
COMPOSITION FOR THE ACCELERATED WOUND HEALING OF DAMAGED TISSUE
COMPOSITION DESTINÉE À LA CICATRISATION ACCÉLÉRÉE DE PLAIES DE TISSU LÉSÉ

(30) Priorität: 19.02.2013 EP 13155711
(43) Veröffentlichungstag der Anmeldung: 30.12.2015
(73) Patentinhaber: Paul Hartmann AG, 89522 Heidenheim (DE)
(72) Erfinder: SMOLA, Hans, 66121 Saarbrücken (DE)
(74) Vertreter: Keller, Günter
(86) Internationale Anmeldenummer: PCT/EP2014/052096
(87) Internationale Veröffentlichungsnummer: WO 2014/127983

(56) Entgegenhaltungen:
- WO-A1-2013/000382
- WO-A1-2013/024012

## Beschreibung

Die vorliegende Erfindung betrifft eine medizinisch-topische Zusammensetzung zur Wundbehandlung umfassend 0,001 bis 3 Gew.-% Fettsäure-Extrakte aus natürlichen Ölen bezogen auf das Gesamtgewicht der medizinisch-topischen Zusammensetzung, wobei es sich bei den natürlichen Ölen um Leinsamenöl oder Rapsöl handelt.

Salben und Hydrogele sind im Stand der Technik bekannt.

So offenbart EP426422 einen Wundverband, auf dem ein Hydrogelmaterial im Hohlraum eines flexiblen Rückenteils aufgebracht ist.

WO2007014672 offenbart ein Hydrogel mit einem Wassergehalt von mindesten 50 Gew.-% sowie dessen Herstellung und Verwendung in der modernen Wundbehandlung.

WO2011095194 beschreibt eine mehrschichtige Wundauflage mit einer wasserhaltigen Hydrogelmatrix und einer absorbierenden Schicht, sowie die Herstellung und Verwendung der wasserhaltigen Hydrogelmatrix.

Es ist ebenfalls bekannt, Fettsäuren in topische Formulierungen einzubringen.

Die WO2011148247 offenbart hier eine omega3-Fettsäuren enthaltende stabilisierende Formulierung zur topischen Anwendung. in Kombination mit Vitamin E, Vitamin C, Pflanzenextrakten und fettlöslichen Antioxidantien.

Die WO2012059158 beschreibt ein Gemisch aus omega3-Fettsäure und einem Inhibitor des NF-kB Transkriptionsfaktors. Das Gemisch eingebunden in eine pharmazeutische Formulierung eignet sich zur Vorbeugung und Behandlung von Entzündungen.

Die US 2008/0193393 offenbart ein Verabreichungssystem für topisch aufgetragene Komponenten, wobei dieses System zwingend eine Fettsäure, ein Phospholipid und ein Öl in einem bestimmten Verhältnis von Gewichtsprozenten zueinander enthält.

Die WO2013000382 offenbart eine pharmazeutische Zubereitung zusammengesetzt aus einem Fettsäure-Gemisch, Pflanzenextrakt und modifizierten Derivaten davon zur Regeneration verletzter Haut, Muskelgewebe und Schleimhäute.

In der nicht vorveröffentlichten internationalen Anmeldung WO 2013/024012 mit der Anmeldenummer PCT/EP2012/065658 wird eine Wirkstoffkombination für die Pflege der Altershaut beschrieben, die aus folgenden Komponenten besteht.
a) 0,01 bis 20 Gew.-% Mandelöl,
b) 0,01 bis 1,5 Gew.-% Leinsamenölfettsäuren,
c) 0,001 bis 1,5 Gew.-% Aminosäuren und
d) 0,01 bis 1,5 Gew.-% Kreatin.

Die dort beschriebenen Wirkstoffkombinationen werden als Wasser-in-ÖI-Emulsionen (w/o), Wasser-in-ÖI-in-Wasser-Emulsionen oder als Schäume formuliert.

Es besteht jedoch weiterhin ein Bedarf an einfach anzuwendenden Formulierungen, die insbesondere die Wundheilung beschleunigen. Die Formulierungen sollen einfach herstellbar, stabil und lagerfähig sein. Es besteht insbesondere ein Bedarf nach wirksamen Formulierungen, deren Wirkstoffe aus natürlich vorkommenden Quellen (insbesondere Pflanzen) stammen.

Dieser Bedarf wird durch den Gegenstand der Patentansprüche erfüllt.

Die Lösung der vorstehenden Aufgabe beruht auf dem überraschenden Befund, dass Fettsäure-Extrakte aus den natürlichen Ölen, Leinsamenöl und Rapsöl eine beschleunigte Wundheilung, insbesondere eine beschleunigte Zellmigration während der Epithelisierungsphase zeigen, insbesondere wenn sie in Form einer Salbe oder eines wasserhaltigen Hydrogels vorliegen und auf eine Wunde aufgebracht werden.

In der vorliegenden Erfindung werden damit Fettsäure-Extrakte aus natürlichen Ölen, ausgewählt aus Leinsamenöl und Rapsöl in einer medizinisch-topischen Zusammensetzung insbesondere in Form einer Salbe oder eines wasserhaltigen Hydrogels zur Verfügung gestellt, die insbesondere zur Wundbehandlung eingesetzt werden können. In einer bevorzugten Ausführungsform wird eine Vorrichtung zur Verfügung gestellt, bei der die medizinisch-topische Zusammensetzung auf einer Wundauflage aufgetragen vorliegt. Die Wundauflage der Vorrichtung unterstützt aufgrund ihrer sekretbindenden oder sekretdurchlässigen Materialeigenschaften die Wundheilung.

Die medizinisch-topischen Zusammensetzungen werden bevorzugt zur Wundbehandlung eingesetzt, wobei während der Epithelisierungsphase die Zellmigration beschleunigt wird.

Unter dem Begriff Wunde werden jegliche mit Gewebezerstörung und Gewebeverlust einhergehende Zusammenhangstrennungen von Gewebe definiert. Der Verschluss eines Gewebedefektes erfolgt zunächst durch eine Gewebereaktion, die unter dem Begriff Wundheilung zusammengefasst werden. Die drei Formen der Wundheilung sind die epitheliale, primäre und sekundäre Wundheilung. Epitheliale Wunden treten bei oberflächlichen Schädigungen des Gewebes auf, wie zum Beispiel Verbrennungen, Verbrühungen, Schürf- oder Risswunden. Primäre Wunden werden beispielsweise durch plastische oder kosmetische Eingriffe wie Nagelextraktion, Phimoseoperation und Hauttransplantationen hervorgerufen. Zu den sekundären Wunden zählen alle infektionsgefärdeten, großflächigen Gewebedefekte, bei der eine oder mehrere Erkrankungen zugrunde liegen können.

Bei der Wundheilung werden drei ineinander übergreifende, zeitlich überlappende Phasen durchlaufen, diese sind die Entzündungs-, Granulations- und Epithelisierungsphase. Bei tieferen Wunden wird während der Entzündungsphase die blutstillende Kaskade unter Einbeziehung der Blutplättchen und Endothelzellen aktiviert. Weiterhin wird unter anderem während der Entzündungsphase ein Fibrinnetz gebildet, das dem neugebildeten Granulationsgewebe als Matrix dient. Während der Granulationsphase wandern Fibroblasten aus dem umliegenden Gewebe in die Wunde ein und bilden ein neues Bindegewebe. Die Fibroblasten produzieren eine Vorstufe des Kollagens, die außerhalb der Zellen unter dem Einfluss verschiedener biochemisch wirksamer Substanzen zu festen Kollagenfasern ausreifen. Die Epithelisierungsphase bringt die Wundheilung schließlich zum Abschluss, dabei kommt es zur Umbildung des Defektgewebes. Die Keratinozyten, die zur Mitose befähigten Zellen aus der Epidermis vermehren sich und beginnen, meist vom Wundrand her, mit der Überhäutung der Granulationsfläche. Das Einwandern der Keratinozyten vom Wundrand her wird als Migration bezeichnet.

Die Wundheilung ist ein dynamisches Geschehen aus katabolischen und anabolischen Prozessen und findet erst bei einer Temperatur von 28°C statt, da ab dieser Temperatur die Mitose erfolgt. Ein feucht-warmes Milieu begünstigt die Wundheilung.

Im Sinne der vorliegenden Erfindung sind die Begriffe "Wundheilung" und "Wundbehandlung" austauschbar.

In der vorliegenden Erfindung konnte gezeigt werden, dass die Fettsäure-Extrakte aus den natürlichen Ölen wie Leinsamenöl oder Rapsöl einen beschleunigenden Effekt auf die Zellmigration während der Epithelisierungsphase ausüben.

Die erfindungsgemäßen Produkte beschleunigen damit die Wundheilung. Da die einzelnen Phasen der Wundheilung zeitlich überlappen, kann die medizinisch-topische Zusammensetzung der vorliegenden Erfindung sowohl für die Behandlung epithelialer als auch primärer und sekundärer Wunden eingesetzt werden.

Die Fettsäure-Extrakte der vorliegenden Erfindung stammen aus natürlichen Ölen. Bei Fettsäure-Extrakten aus natürlichen Ölen handelt es sich um freie Fettsäuren. Die freien Fettsäuren sollen die Wunde, das geschädigte Gewebe mit essentiellen Fettsäuren versorgen, dabei soll die Freisetzung aus Triglyceriden aus Gründen der Bioverfügbarkeit umgangen werden. Die Fettsäure-Extrakte aus natürlichen Ölen gemäß der Erfindung liegen damit bevorzugt in Form freier Fettsäuren und nicht als Triglycerid vor. Die natürlichen Öle werden für die pharmazeutische und kosmetische Industrie durch Kaltpressung gewonnen. Je nach Art und Herkunft der natürlichen Produkte für die Ölgewinnung und dem Verfahren der Ölgewinnung schwankt die natürliche Zusammensetzung der Öle und der daraus gewonnenen Fettsäuren.

Medizinisch-topische Zusammensetzungen, die eine Wirkstoffkombination enthalten, die aus folgenden Komponenten bestehen
a) 0,01 bis 20 Gew.-% Mandelöl,
b) 0,01 bis 1,5 Gew.-% Leinsamenölfettsäuren,
c) 0,001 bis 0,5 Gew.-% Aminosäuren und
d) 0,01 bis 1,5 Gew.-% Kreatin
sind ausgeschlossen.

Die natürlichen Öle, Leinsamenöl, Rapsöl, Sojaöl und Olivenöl dienen als Ausgangssubstanz für die Isolierung der Fettsäure-Extrakte. Natürliche Öle haben folgende Fettsäure-Zusammensetzung (Zahl der C-Atome: Zahl der Doppelbindungen in Klammern) in den jeweils angegebenen relativen Anteilen:

| | Leinsamenöl | Rapsöl | Sojaöl | Olivenöl |
|---|---|---|---|---|
| Palmitinsäure (C16:0) | 4-8 % | 2-7 % | 4-8 % | 11-15 % |
| Stearinsäure (C18:0) | 2-5 % | 1-3 % | 4-7 % | 1-3 % |
| Ölsäure (C18:1) | 16-26 % | 55-65 % | 25-35 % | 55-71 % |
| Linolsäure (C18:2) | 14-24 % | 19-31 % | 52-68 % | 13-23 % |
| Linolensäure (C18:3) | 43-55 % | 8-10 % | 5-8 % | 1-3 % |

sowie Fettsäuren mit Kohlenstoffketten, die mehr als 18 Kohlenstoffatome aufweisen 0,1-2 %; wobei sich die Prozentangaben zu 100 % addieren.

Erfindungsgemäß wurde gefunden, dass Extrakte aus Leinsamenöl und Rapsöl, insbesondere aus Leinsamenöl, die Wundheilung wesentlich stärker beschleunigen, als Extrakte aus anderen natürlichen Ölen, wie Sojaöl und Olivenöl.

In einer bevorzugten Ausführungsform handelt es sich bei den natürlichen Ölen um Leinsamenöl mit folgender Fettsäure-Zusammensetzung:
Palmitinsäure 4-8 %,
Stearinsäure 2-5 %,
Ölsäure 16-26 %,
Linolsäure 14-24%,
Linolensäure 43-55 % sowie Fettsäuren mit Kohlenstoffketten, die mehr als 18 Kohlenstoffatome aufweisen 0,1-2 % und wobei sich die einzelnen Komponenten auf 100 % addieren.

In einer bevorzugten Ausführungsform werden die Fettsäure-Extrakte aus Leinsamenöl oder Rapsöl durch alkalische Hydrolyse mit anschließender Ansäuerung gewonnen. In einer besonders bevorzugten Ausführungsform werden die Fettsäure-Extrakte aus Leinsamenöl durch alkalische Hydrolyse mit anschließender Ansäuerung gewonnen.

In einer anderen Ausführungsform werden die Fettsäure-Extrakte aus Leinsamenöl oder Rapsöl, besonders bevorzugt aus Leinsamenöl, durch Hydrolyse, anschließender Ansäuerung und anschließender Destillation gewonnen.

In der vorliegenden Erfindung konnte in einer *in vitro* Rekonstruktion humaner Epidermis gezeigt werden, dass sich die Fettsäure-Extrakte aus Leinsamenöl (omega 6 zu omega3: 1:2 bis 1:4) und die Fettsäure-Extrakte aus Rapsöl (2:1 bis 4:1) im Vergleich zu Fettsäure-Extrakte aus Sojaöl (7:1) oder Olivenöl (4:1) beschleunigend auf die Wundheilung auswirken. Die in Klammern wiedergegebenen Zahlenwerte bezeichnen das Verhältnis von omega6 zu omega3 Fettsäuren.

In der vorliegenden Erfindung wird die beschleunigte Wundheilung geschädigten Gewebes an einer *in vitro* Rekonstruktion humaner Epidermis mittels der zeitaufgelösten Impedanzmessung gezeigt, nach der *in vitro* Änderungen der Zellmorphologie für bioanalytische Zwecke genutzt werden können. Zunächst werden für die impedimetrische Messung Zellen auf einer flachen Goldfilm-Elektrode kultiviert. Anschließend wird die Impedanz (Wechselstromwiderstand) der zellbedeckten Elektrode bei einer oder mehreren Frequenzen als Funktion der Zeit gemessen. Die Zellen verhalten sich aufgrund der isolierenden Eigenschaften ihrer Plasmamembran wie dielektrische Partikel. Mit zunehmender Zelldichte nimmt die Impedanz zu. Nachdem die Elektrode mit einer kontinuierlichen Zellschicht bedeckt ist, wird die gemessene Impedanz maßgeblich durch die Zellmorphologie bestimmt. Ändert sich die Zellform infolge eines externen Stimulus, so ändern sich die Stromwege um und durch die Zelle, so dass die gemessene Impedanz sich entsprechend verändert.

Maßgeblich wird die Wundheilung durch Fettsäure-Extrakte aus Leinsamenöl und durch Fettsäure-Extrakte aus Rapsöl beschleunigt, insbesondere durch Fettsäure-Extrakte aus Leinsamenöl. Der beschleunigende Effekt auf die Wundheilung wird in einer *in vitro* Rekonstruktion humaner Epidermis nachgewiesen. Dafür werden Keratinozyten (HACAT-Zellen) auf einer Goldelektrode kultiviert, mit den Fettsäure-Extrakten aus den natürlichen Ölen versetzt, einem elektrischen Verwundungspuls ausgesetzt und impedimetrisch untersucht. In den Wundheilungs-Assays erwiesen sich überraschenderweise die Fettsäure-Präparationen aus Rapsöl, insbesondere Fettsäure-Extrakte aus Leinsamenöl beschleunigend auf die Migrationsleistung der Keratinozyten. Die Einflüsse der Fettsäure-Extrakte aus Olivenöl und Sojaöl blieben jedoch weitgehend unauffällig.

Zunächst wurde in einem ersten experimentellen Ansatz mittels impedimetrischen Methoden zur elektrischen Charakterisierung der intakten Keratinozyten der Einfluss verschiedener Fettsäure-Extrakte auf eine mögliche Modulation der epithelialen Barrierefunktion der Zellen untersucht. Hierbei nehmen die Fettsäure-Extrakte aus Leinsamenöl, Olivenöl, Rapsöl und Sojaöl nach einer Inkubationszeit von 24 oder 32 Stunden keinerlei Einfluss auf die Barrierefunktion intakter Zellen. Es ist weder eine signifikante Verstärkung der epithelialen Barriere durch die Fettsäuren noch eine Reduktion derselben zu verzeichnen.

Anschließend wurde der Einfluss der Fettsäure-Extrakte aus natürlichen Ölen auf die beschleunigte Wundheilung am Beispiel eines *in vitro* Modells an Keratinozyten mittels histologischer Validierungsexperimente untersucht. Die Keratinozyten wurden dabei ebenfalls auf einer Goldelektrode kultiviert, mit Fettsäure-Extrakten aus natürlichen Ölen versetzt und anschließend einem elektrischen Verwundungspuls ausgesetzt. Die Unterscheidung lebender und toter Zellen basiert auf Fluoreszenzfarbstoffen. Als Marker für lebende Zellen dient das membrangängige Fluorophor-Precursor Calcenin AM (CAM), das durch unspezifische Esterasen aus dem Zytoplasma zu grün fluoreszierendem Calcein umgesetzt wird. Als Marker toter Zellen wird hingegen der DNA Interkalationsfarbstoff Ethidium-Homo-Dimer (ETHD), der die Plasmamembran vitaler Zellen nicht überwinden kann, eingesetzt. Tote Zellen sind aufgrund ihrer intensiv roten fluoreszierender Kerne von den lebenden Zellen zu unterscheiden. Damit ergibt sich zu jedem gewünschten Zeitpunkt des Assays ein fluoreszenzmikroskopisch leicht zu interpretierendes Bild über die Verteilung von lebenden und toten Zellen. Zwei respektive vier Stunden nach der elektrischen Verwundung lässt sich das fortschreitende Einwachsen der Zellen (Migration) aus der Peripherie deutlich erkennen. Tote Zellen mit roten Kernen verschwinden von den Elektroden und werden durch die aus der Peripherie eingewanderten Zellen mit grün leuchtendem Zytoplasma ersetzt.

Basierend auf impedimetrischen Messungen wurde ferner die optimale Konzentration der eingesetzten Fettsäure-Extrakten aus natürlichen Ölen bestimmt.

In einer bevorzugten Ausführungsform beträgt der Anteil an Fettsäure-Extrakt aus den natürlichen Ölen in der medizinisch-topischen Zusammensetzung zur Wundbehandlung 0,005 bis 1,5 Gew.-%, bevorzugt 0,015 bis 0.3 Gew.-% bezogen das Gesamtgewicht der medizinisch-topischen Zusammensetzung.

In einer besonderen Ausführungsform ist die medizinisch-topische Zusammensetzung zur Wundbehandlung eine Salbe.

Eine Salbe im Sinne der vorliegenden Erfindung, ist eine halbfeste und homogen aussehende Zubereitung und dient der lokalen Applikation der Fettsäure-Extrakte aus den natürlichen Ölen. Halbfeste Zubereitungen zur kutanen Anwendung bestehen aus einer einfachen oder zusammengesetzten Grundlage, in der in der Regel ein Wirkstoff oder mehrere Wirkstoffe gelöst oder dispergiert sind. Unter dem Begriff "Salben" fallen folgende Darreichungsformen wie einphasiges lipophiles System, Gele wie zum Beispiel Hydrogele, Cremes und Pasten. Salben sind bekannt und in Standardwerken wie z.B. von Schöffling (Arzneiformenlehre: Ein Lehrbuch der Galenik für Theorie und Praxis, 5. Auflage (2009)) und von Bauer, Frömming und Führer (Pharmazeutische Technologie 9. Auflage (2012)) beschrieben.

In einer weiteren bevorzugten Ausführungsform handelt es sich bei der Salbe um ein einphasiges lipophiles System oder um ein wasserhaltiges Hydrogel. Das lipophile System besteht aus einer lipophilen Grundlage natürlicher und/oder synthetischer Stoffe.

In einer bevorzugten Ausführungsform der Erfindung enthält das einphasige lipophile System mindestens einen Bestandteil ausgewählt aus der Gruppe umfassend Na-Carboxymethylcellulose, amorphes Siliciumdioxid, teilweise oder vollständig verestertes Glycerin, Polyethylenglykol, ethoxylierte höhere Alkohole, natürliche oder synthetische Wachse, Paraffin, Gemische aus höheren aliphatischen Alkanen, Ester aus Benzoesäure, Zimtsäure oder Benzylalkohol und Mischungen davon.

In einer bevorzugten Ausführungsform enthält das einphasige lipophile System als weiteren Salbenbestandteil Glycerin, das teilweise oder vollständig mit einem Gemisch aus Bernsteinsäure und Isostearinsäure, einem Gemisch aus Stearinsäure und Palmitinsäure, einem Gemisch aus Caprylsäure und Caprinsäure, einem Gemisch aus gesättigten pflanzlichen C₈ bis C₁₈ Fettsäuren und Kombinationen davon, verestert ist.

In einer besonders bevorzugten Ausführungsform enthält das einphasige lipophile System als weiteren Bestandteil Paraffin oder vollständig mit gesättigten pflanzlichen C₈ bis C₁₈ Fettsäuren verestertes Glycerin.

Besonders bevorzugt sind erfindungsgemäß einphasige lipophile Systeme, die alle zwei vorstehend genannten Salbenbestandteile enthalten.

Die erfindungsgemäßen Salben enthalten in der Regel weitere Inhaltsstoffe, wie sie für Salben üblich sind. Dazu zählen beispielhaft Ölkomponenten, Emulgatoren, Lösungsvermittler, Konservierungsmittel, Antioxidantien, Viskositäts- und pH-Regulierer, Farb- und Duftstoffe, sowie Wasser. Die Herstellung der erfindungsgemäßen Salben erfolgt auf an sich bekannte Art und Weise, wie sie in Standardwerken wie z.B. von Schöffling (Arzneiformenlehre: Ein Lehrbuch der Galenik für Theorie und Praxis, 5. Auflage (2009)) beschrieben ist.

Wasserhaltige Hydrogele setzen sich aus wasserunlöslichen Polymeren zusammen, die sich zu einem dreidimensionalen Netzwerk verknüpfen. Sie bestehen zu 40 - 90 Gew.-% aus gebundenem Wasser oder wässriger Lösung. Als wässrige Lösung können beispielsweise Lösungen mit einem definierten pH-Wert und Elektrolytspektrum, wie zum Beispiel die Ringerlösung eingesetzt werden.

Wasserhaltige Hydrogele unterstützen aufgrund ihres hohen Feuchtigkeitsgehaltes die Wundheilung. Das wasserhaltige Hydrogel in der vorliegenden Erfindung wird für die lokale Verabreichung der Fettsäure-Extrakte aus natürlichen Ölen verwendet. Bei den wasserhaltigen Hydrogelen wird der wundheilende Effekt der erfindungsgemäß eingesetzten Fettsäure-Extrakte überraschenderweise deutlich verstärkt.

Bei einer bevorzugten erfindungsgemäßen Ausführungsform handelt es sich um ein wasserhaltiges Hydrogel auf Polysaccharid-Basis. Derartige Hydrogele sind z.B. in der Patentliteratur WO2007014672 beschrieben, auf die insoweit Bezug genommen wird.

Bei dieser Ausführungsform umfasst das wasserhaltige Hydrogel mindestens ein gelbildendes Polymer und mindestens einen mehrwertigen Alkohol, wobei das gelbildende Polymer ein Polysaccharid ist.

Bei dieser Ausführungsform ist weiter bevorzugt, dass das wasserhaltige Hydrogel mindestens ein gelbildendes Polymer, mindestens einen mehrwertigen Alkohol und mindestens ein Acrylsäurederivat, wobei das gelbildende Polymer ein Polysaccharid ist, umfasst.

Bei dieser Ausführungsform ist das gelbildende Polysaccharid bevorzugt aus der Gruppe umfassend Hydroxycellulose, Carboxycellulose, Stärke, Alginate oder deren Derivate, Chitin oder dessen Derivate oder Salze ausgewählt.

Bei dieser Ausführungsform ist der mehrwertige Alkohol bevorzugt aus der Gruppe umfassend Glycerin, Sorbit, Polypropylenglykol und Polyethlyenglykol ausgewählt.

Bei dieser Ausführungsform ist das Acrylsäurederivat bevorzugt eine Polyacrylsäure.

Bei einer weiteren bevorzugten Ausführungsform handelt es sich um ein wasserhaltiges Hydrogel, das basierend auf einer Mischung aus einem Vorpolymer mit Isophorondiisocyanatendgruppen und einem Diamin auf Polyethylenoxidbasis basiert. Derartige Hydrogele sind beispielsweise in der Patentliteratur EP426422 und WO2011095194 beschrieben. Bei dieser bevorzugten Ausführungsform handelt es sich um wasserhaltiges Hydrogel, wobei das wasserhaltige Hydrogel mindestens ein gelbildendes Polymer und mindestens einen mehrwertigen Alkohol umfasst und wobei das gelbildende Polymer eine Mischung aus einem Vorpolymer mit Isophorondiisocyanatendgruppen und einem Diamin auf Polyethylenoxidbasis ist, wobei das Verhältnis der reaktiven Gruppen des Isocyanats zu den Amidgruppen des Diamins bevorzugt bei 1:1.25 bis 1:1.35 liegt.

Das Vorpolymer mit den Isophorondiisocyanatendgruppen ist eine Vorstufe des Gel-Polymers, das nach erfolgter Reaktion mit einem Diamin erhalten wird. Zur Ausbildung eines dreidimensionalen Gel-Netzwerkes liegt das Verhältnis ("NCO" : "NH2") der reaktiven Gruppen des Isocyanats ("NCO") des Vorpolymers zu den reaktiven Gruppen des Diamins ("NH2") bevorzugt bei 1:1.25 bis 1:1.35.

Beispielsweise kann als ein Diamin auf Polyethylenoxidbasis das Produkt Jeffamine ED-2003 und als ein Vorpolymer mit Isophorondiisocyanatendgruppen das Produkt Aquapol PI-13000-3 eingesetzt werden.

In einer bevorzugten Ausführungsform handelt es sich erfindungsgemäß um ein wasserhaltiges Hydrogel, das als Lösungsvermittler ein Tensid enthält.

Tenside wirken als Lösungsvermittler, indem sie beispielsweise die Grenzflächenspannung zwischen zwei Phasen herabsetzen.

In einer besonders bevorzugten Ausführungsform handelt es sich um ein wasserhaltiges Hydrogel, das als Lösungsvermittler ein Tensid enthält ausgewählt aus der Gruppe umfassend Natriumlaurylsulfat, Octoxinol-9, Polysorbat 20, Salze der Gallensäuren und Octylglucosid.

Erfindungsgemäß wird ebenfalls eine Vorrichtung zur Verfügung gestellt, bei der die erfindungsgemäße medizinisch-topische Zusammensetzung auf einer Wundauflage aufgebracht ist.

Eine Wundauflage wird auf eine äußere Wunde gelegt, sie verhindert das Eindringen von Fremdkörpern in die Wunde und verhindert das Verkleben der Wunde mit der Kleidung. Abhängig vom verwendeten Material weisen Wundauflagen sekretbindende oder sekretdurchlässige Eigenschaften auf.

Geeignete Wundauflagen sind z.B. in der Modernen Wundversorgung von Protz (6. Auflage (2011)) beschrieben und enthalten ein Fasermaterial oder ein Polymer, ausgewählt aus Baumwolle, Viskose, Polyamid, Polyurethan und/oder Polyester.

Wundauflagen mit Baumwollfasern bestehen in der Regel aus einem Gewebe aus Baumwollfasern. Baumwollfasern bestehen neben einer Wachsschicht fast ausschließlich aus hochkristalliner Zellulose. Die besondere Anordnung der Zellulose verleiht der Baumwolle eine hohe Reißfestigkeit. Baumwollgewebe, mit einer fein- oder grobmaschigen Gitterstruktur ist sehr saugfähig und kann bis zu 65% seines Gewichtes an Wasser und somit auch an Wundexsudat aufnehmen. Baumwollgewebe gelten als sehr hautfreundlich und besitzen ein äußerst geringes Allergiepotential.

Viskosefasern sind Chemiefasern, die vom Grundstoff Zellulose ausgehend mit Hilfe des Viskoseverfahrens industriell hergestellt werden. Zellstoff als Ausgangsmaterial für Viskosefasern stammt beispielsweise aus Holz von Buchen, Fichten, Eukalyptus, Pinien oder Bambus. Die chemische Zusammensetzung ähnelt der von Baumwolle. Bei der Herstellung der Viskosefasern werden Sägespäne mit Chemikalien gekocht, um die Cellulose herauszulösen. Anschließend wird die Cellulose mit Wasser, Natronlauge und Schwefelkohlenstoff zu einem Viskosebrei verarbeitet und durch Spinndüsen zu Viskosegarn verfestigt.

Polyamide sind teilkristalline thermoplastische Polymere, deren Eigenschaften weitgehend durch die Amidgruppen dominiert werden, die über Wasserstoffbrückenbindungen miteinander wechselwirken. Die Eigenschaften der Polyamide hängen unter anderem von deren kristallinem Gefüge und insbesondere von deren Wassergehalt ab. Polyamide können Wassermoleküle reversibel auf- und abgeben, dabei wird das Wasser in die amorphen Bereiche des Polyamids eingelagert. Die Fähigkeit zur Wasseraufnahme hängt dabei wesentlich von der Konzentration der Amidgruppen ab.

Polyurethane sind Kunststoffe, die aus der Polyadditionsreaktion mehrwertiger Alkohole mit Polyisocyanaten entstehen. Abhängig von ihrer Herstellung können sie hart und spröde oder weich und elastisch sein. Polyurethan in Form eines dauerelastischen Weichschaums kann ebenfalls als Wundauflage eingesetzt werden. Aufgrund der Kapillarwirkung des grobporigen Schaumes verfügen Wundauflagen auf Polyurethanbasis über ein gutes Feuchtetransportvermögen. Beispielsweise nimmt Polyurethan-Schaumstoff in Kombination mit wasserhaltigen Hydrogelen Wundexsudat auf, ohne die Wunde auszutrocknen.

Polyester sind überwiegend synthetische Polymere mit einer Esterfunktion in ihrer Hauptkette. Polyesterfasern können allerdings im Vergleich zu Baumwoll-, Viskose- und Polyamidfasern kein Wasser aufnehmen. Sie verfügen über ein gutes Feuchtetransportvermögen und trocknen schnell. Mischungen aus Polyester und Baumwolle können ebenfalls als Wundauflagen eingesetzt werden.

In einer bevorzugten Ausführungsform der erfindungsgemäßen Vorrichtung zur Wundbehandlung umfasst die Wundauflage ein oder mehrere Materialien ausgewählt aus Polyamid, Viskose und Baumwolle und weist sekretbindende Eigenschaften auf.

In einer weiter bevorzugten Ausführungsform der erfindungsgemäßen Vorrichtung zur Wundbehandlung umfasst die Wundauflage ein oder mehrere Materialien ausgewählt aus Polyester und Polyurethan und weist sekretdurchlässige Eigenschaften auf.

In einer bevorzugten Ausführungsform ist die erfindungsgemäße Vorrichtung zur Wundbehandlung mit elementarem Silber und/oder mit Aktivkohle beschichtet.

Die Wirkmechanismen der Silber-Ionen beruhen auf der Blockierung von Enzymen, Unterbindung lebensnotweniger Transportfunktionen und der Schädigung der Membranstruktur.

In einer weiteren bevorzugten Ausführungsform liegt das wasserhaltige Hydrogel in einer Dosier-Spritze vor und wird gegebenenfalls direkt in eine Wundkavität appliziert oder auf eine Wundauflage aufgespritzt.

Mittels einer Dosier-Spritze ist ein absolut zielgerichtetes Aufbringen des wasserhaltigen Hydrogels auf oder in oberflächliche oder tiefe Wunden gewährleistet. Dabei wird die gewünschte Menge des Gels auf die gewünschte Stelle der Wunde appliziert. Diese Applikationsform ist besonders geeignet, wenn nur bestimmte Bereiche der Wunde mit dem erfindungsgemäßen Gel behandelt werden.

In einer erfindungsgemäß bevorzugten Vorrichtung ist die medizinisch-topische Zusammensetzung zur Wundbehandlung ein einphasiges lipophiles System, das gleichmäßig auf der Wundauflage verteilt ist, in einer Menge an 100-350 g einphasiges lipophiles System / m² Wundauflage, bevorzugt 125-300 g einphasiges lipophiles System / m² Wundauflage, weiter bevorzugt 150-270 g einphasiges lipophiles System / m² Wundauflage.

In einer erfindungsgemäßen Ausführungsform wird die medizinisch-topische Zusammensetzung zur Wundbehandlung oder die Vorrichtung zur Wundbehandlung zur Verwendung zur beschleunigten Zellmigration während der Epithelisierungsphase.

Die Erfindung wird durch die nachfolgenden Beispiele näher erläutert, wobei die Komponenten handelsübliche Ausgangsmaterialien für medizinisch-topische Zusammensetzungen sind.

### Beispiel 1

### Extraktion der Fettsäuren aus natürlichen Ölen

In einem 11-Erlenmeyerkolben werden 250 ml Glycerin (99 %) und 40 g Kaliumhydroxid (0,71 mol) vorgelegt. Die Lösung wird auf 120-140°C erhitzt und geschwenkt bis das Kaliumhydroxid vollständig gelöst ist. Zu der heißen Lösung wird 110 ml des auf 110-115°C vorgeheizten Öls gegeben. Dies wird für Rapsöl, Leinsamenöl, Sojaöl und Olivenöl durchgeführt. Die Lösung wird weiter erhitzt und stark gerührt, bis die Verseifung vollständig ist. Die Vollständigkeit der Reaktion zeigt sich in der Schaumbildung. Nach dem Abkühlen der Mischung werden vorsichtig 150 ml Schwefelsäure (25 % V/V) unter Rühren zugegeben. Danach werden 200 ml Wasser zugegeben. Die entstehenden Phasen werden separiert. Die Fettsäurephase wird zwei Mal mit je 500 ml heißem Wasser gewaschen. Die Fettsäuren werden durch einen großen beheizten Hirsch-Trichter filtriert und anschließend bei 130°C unter starkem Rühren getrocknet.

### Beispiel 2

### Fettsäure-Extrakt/BSA- Lösung

Die isolierten Fettsäure-Extrakte aus Beispiel 1 werden auf 37°C vorgewärmt, anschließend in einem stöchiometrischen Verhältnis von 1:1 mit 96 %-igen Ethanol vermischt (FS-Stocklösung). 1,34 g delipidiertes BSA wird in 10 ml serumfreiem Medium gelöst. Eine 2 mM Stammlösung setzt sich aus 2,3 µl FS-Stocklösung in 2 ml BSA-Stocklösung zusammen. Für die Wundheilungsassays wird die Stammlösung in serumfreien Medium entsprechend verdünnt und sterilfiltriert.

### Beispiel 3

### Wundheilungsassay

Im Rahmen der vorliegenden Erfindung wurde ein *in vitro-*System verwendet, bei dem menschliche primäre Keratinozyten (HACAT-Zellen) auf einer flachen Gold-Elektrode (Länge: 650 µm, Breite: 175 µm) zur impedimetrischen Messung gezüchtet wurden. Hierfür wurden das übliche Standardnährmedium (BIOCHROM) mit entsprechenden Wachstumsfaktoren und Zusatzstoffen zugesetzt. Das Medium setzt sich aus DMEM/high glucose, 10 % FCS, 1 % Penicillin / Streptomycin und 1 % L-Glutamin zusammen. Danach wurden die Zellen mit 100 µM, 500 µM bzw. 1000 µM Fettsäure-Extrakt aus natürlichen Ölen und delipidierten BSA als Lösungsvermittler (1:1 Stöchiometrie) in serumfreien Medium - zum möglichst vollständigen Entzug aller Wachstumsfaktoren und Mitogene - für 24 respektive 32 Stunden vorinkubiert. Zur Kontrolle wurden Messungen durchgeführt, bei denen die Zellen ausschließlich mit serumfreien Medium und unbeladenem, delipidiertem BSA inkubiert wurden. Anschließend wurden die Zellen mit einem elektrischen Puls von 30 Sekunden bei einer Frequenz von 32 kHz, einer applizierten Stromstärke von 2500 µA lokal abgetötet. Danach wird die Migrationsfähigkeit der Zellen aus der Peripherie der Elektrode bei einer Meßfrequenz von 32 kHz verfolgt.

Impedimetrische Wundheilungsexperimente in Gegenwart der aus Leinsamenöl extrahierten Fettsäuregemische (Fettsäure-Extrakt aus Leinsamenöl) zeigen einen nicht signifikanten Einfluss auf die Migrationsfähigkeit der Keratinozyten unter Einfluss von 100 µM Fettsäure-Extrakt aus Leinsamenöl, jedoch eine signifikant beschleunigende Migrationsfähigkeit unter Einfluss von 500 µM bzw. 1000 µM Leinölextraktes.

500 µM Fettsäureextrakte aus Rapsöl zeigen eine tendenziell beschleunigte, jedoch nicht signifikante Zellmigration in impedimetrischen Wundheilungsexperimenten, jedoch einen signifikant beschleunigten Einfluss der Zellmigration bei 1000 µM Fettsäure-Extrakte aus Rapsöl.

Impedimetrische Wundheilungsexperimente in Gegenwart der aus Sojaöl bzw. Olivenöl extrahierten Fettsäureextrakte zeigen keine signifikant verbesserte Migrationsfähigkeit der Keratinozyten unter Einfluss von 100 µM, 500 µM bzw. 1000 µM Sojaöl- bzw. Olivenölextrakten.

### Beispiel 4

### Rezeptur 1 : Medizinisch-topische Zusammensetzung auf Paraffinbasis (einphasiges lipophiles System)

In einem Homogenisierer des Unternehmens Fryma (Homogenisierer) werden 0.015-0.3 Gew.-% Fettsäureextrakt aus Leinsamen mit 99.7-99.985 Gew.-% Paraffin (einem Gemisch hauptsächlich höherer aliphatischer Alkane) auf 85°C erwärmt. Bei einer Rührwerkeinstellung von 10 U/Min. wird die Temperatur etwa 15 Minuten gehalten.

Die Menge der einzelnen Komponenten wird so eingestellt, dass deren Summe 100 Gew.-% ergibt.

### Beispiel 5

### Rezeptur 2: Medizinisch-topische Zusammensetzung überwiegend auf Triglyceridbasis (einphasiges lipophiles System)

Nacheinander werden 60-70 Gew.-% vollständig mit gesättigten pflanzlichen C₈ bis C₁₈ Fettsäuren verestertes Glycerin und 20-30 Gew.-% teilweise mit verschiedenen Fettsäuren verestertes Diglycerin, und 0.015-0.3 Gew.-% Fettsäure-Extrakt aus Leinsamenöl in einen Homogenisierer des Unternehmens Fryma bei 80°C aufgeschmolzen und dabei homogen gemischt.

Die Menge der einzelnen Komponenten wird so eingestellt, dass deren Summe 100 Gew.-% ergibt

### Beispiel 6

### Rezeptur 3: Medizinisch-topische Zusammensetzung (einphasiges lipophiles System)

In einem Homogenisierer der Firma Fryma werden 0.015-0.3 Gew.-% Fettsäureextrakt aus Rapsöl, 20-30 Gew.-% Paraffin, 15-25 Gew.-% teilweise mit verschiedenen Fettsäuren verestertes Diglycerin, 20-30 Gew.-% vollständig mit gesättigten pflanzlichen C₈ bis C₁₈ Fettsäuren verestertes Glycerin, 2-10 Gew.-% mit Stearinsäure und Palmitinsäure verestertes Glycerin, 2-10 Gew.-% teilweise mit Bernsteinsäure und Isostearinsäure verestertes Glycerin und 15-25 Gew.-% Na-Carboxymethylcellulose auf 80°C erwärmt und dabei bei einer Rührwerkeinstellung von 10 U/Min homogen gemischt.

Die Menge der einzelnen Komponenten wird so eingestellt, dass deren Summe 100 Gew.-% ergibt.

### Beispiel 7

### Rezeptur 4: Medizinisch-topische Zusammensetzung auf Hydroxyethylcellulosebasis (wasserhaltiges Hydrogel)

Das wasserhaltige Hydrogel setzt sich zusammen aus:
0.015-0.3 Gew.-% Fettsäure-Extrakt aus Leinsamenöl
0.1-5 Gew.-% Polyacrylat
10-20 Gew.-% Glycerin
0.5-5 Gew.-% Hydroxyethylcellulose
0.1-5 Gew.-% Na-Carboxymethylcellulose
Rest: gereinigtes Wasser oder gereinigte wässrige Lösung für pharmazeutische Zwecke

Die Menge der einzelnen Komponenten wird so eingestellt, dass deren Summe zusammen mit dem zugesetzten Wasser oder der wässrigen Lösung 100 Gew.-% ergibt.

Zur Herstellung der Hydrogele wird zunächst eine Suspension aus pulverförmiger Hydroxyethylcellulose, Na-Carboxymethylcellulose und Glycerin hergestellt, indem das pulverförmige Polysaccharid langsam in Glycerin gegeben und gleichmäßig verrührt wird. Anschließend wird eine Lösung des Polyacrylats hergestellt. Hierzu wird Polyacrylat in Wasser gegeben und zwei Stunden gerührt. Unter gleichmäßigem Rühren wird sehr langsam die Suspension aus Polysaccharid/Glycerin zur Polyacrylat-Lösung zugegeben und für mindestens zwei weitere Stunden bei Raumtemperatur gerührt. Die bei der Herstellung des Gels eingearbeitete Luft kann durch Rühren im Vakuum wieder entfernt werden. Die resultierenden Gele werden in Tuben gefüllt und können mittels β-Sterilisation sterilisiert werden. Die resultierenden transparenten wasserhaltigen Hydrogele weisen eine gute Modellierbarkeit auf.

### Beispiel 8

### Rezeptur 5: Medizinisch-topische Zusammensetzung auf Basis eines Vorpolymers (wasserhaltiges Hydrogel)

Das wasserhaltige Hydrogel setzt sich zusammen aus:
0,015-0,3 Gew.-% Fettsäureextrakt aus Leinsamenöl
15-30 Gew.-% Polyethylenglykol
8-14 Gew.-% Vorpolymer mit Isophorondiisocyanatendgruppen
5-10 Gew.-% Diamin auf Polyethylenoxidbasis
0-5 Gew.-% anorganisches Chlorid
0-2 Gew.-% Tensid
Rest: gereinigtes Wasser oder wässrige Lösung für pharmazeutische Zwecke

Die Menge der einzelnen Komponenten wird so eingestellt, dass deren Summe zusammen mit dem zugesetzten Wasser oder der wässrigen Lösung 100 Gew.-% ergibt.

### Komponente A:

Zunächst wird Polyethylenglykol mit 45-90 Gew.-% Wasser, gegebenenfalls dem anorganischen Chlorid (chloridhaltige anorganische Verbindung wie bspw. NaCl), gegebenenfalls dem Tensid und dem Fettsäure-Extrakt gemischt und anschließend auf 2°C abgekühlt.

### Komponente B:

Zur Herstellung der wässrigen Komponente B wird zunächst das das Diamin auf Polyethlenoxidbasis bei 50°C aufgeschmolzen, zu 5-10 Gew.-% Wasser gemischt und auf Raumtemperatur abgekühlt.

### Komponente C:

Das Vorpolymer mit Isophorondiisocyanatendgruppen wird auf Raumtemperatur gebracht.

Die Komponenten A, B und C werden im Verhältnis 75.4:14.4:10,6 miteinander homogen vermischt und möglichst blasenfrei in bereitgestellte Formen gegossen.

### Beispiel 9

### Rezeptur 6: Medizinisch-topische Zusammensetzung auf Basis eines Vorpolymers (wasserhaltiges Hydrogel)

Das wasserhaltige Hydrogel setzt sich zusammen aus:
0.015-0.3 Gew.-% Fettsäure-Extrakt aus Leinsamenöl
37-43 Gew.-% Propylenglykol
12-16,5 Gew.-% Vorpolymer mit Isophorondiisocyanatendgruppen und ein Diamin auf Polyethylenoxidbasis
0-5 Gew.-% anorganischen Chlorids
0-2 Gew.-% Tensid,
wobei das Verhältnis der reaktiven Gruppen von Isocyanat zu den Amingruppen des Diamins 1:1.25 bis 1:1.35 betragen soll.
Rest: gereinigte wässrige Lösung für pharmazeutische Zwecke

Die Menge der einzelnen Komponenten wird so eingestellt, dass deren Summe zusammen mit der zugesetzten wässrigen Lösung 100 Gew.-% ergibt.

Das Herstellungsverfahren des Hydrogels erfolgte analog zu Beispiel 9.

### Beispiel 10

### Baumwolle-Wundauflage Imprägnierung

Rezeptur 1 auf Paraffinbasis aus Beispiel 4 wird in einer Menge von 265 g einphasiges lipophiles System / m² Baumwolle-Wundauflageauf einer wahlweise mit Silber und/oder Aktivkohle beschichteten Baumwolle-Wundauflage aufgetragen.

### Beispiel 11

### Polyester Imprägnierung

185 g einphasiges lipophiles System / m² Polyester-Wundauflage der Rezeptur 2 wird auf einen Polyester-Wundauflage gleichmäßig verteilt.

### Beispiel 12

### Polyamid-Wundauflage Imprägnierung

Rezeptur 3 wird in einer Menge von 210 g einphasiges lipophiles System / m² Polyamid-Wundauflage auf eine wahlweise mit Silber und/oder Aktivkohle beschichtete Polyamid-Wundauflage gleichmäßig verteilt.

### Beispiel 13

### Polyurethan Imprägnierung

Das wasserhaltige Hydrogel aus der bereitgestellten Form aus Rezeptur 6 wird mit einem Druck von 200 N/m² auf Polyurethanschaum-haltige Wundauflage aufgebracht.

## Patentansprüche

1. Medizinisch-topische Zusammensetzung zur Wundbehandlung umfassend 0,001 bis 3 Gew.-% Fettsäure-Extrakte aus natürlichen Ölen bezogen auf das Gesamtgewicht der medizinisch-topischen Zusammensetzung, **dadurch gekennzeichnet, dass** es sich bei den natürlichen Ölen um Leinsamenöl oder Rapsöl handelt, wobei medizinisch-topische Zusammensetzungen, die eine Wirkstoffkombination enthalten, die aus folgenden Komponenten bestehen
a) 0,01 bis 20 Gew.-% Mandelöl,
b) 0,01 bis 1,5 Gew.-% Leinsamenölfettsäuren,
c) 0,001 bis 0,5 Gew.-% Aminosäuren und
d) 0,01 bis 1,5 Gew.-% Kreatin
ausgeschlossen sind.

2. Medizinisch-topische Zusammensetzung zur Wundbehandlung nach Anspruch 1, **dadurch gekennzeichnet, dass** es sich bei der medizinisch-topischen Zusammensetzung um eine Salbe handelt.

3. Medizinisch-topische Zusammensetzung zur Wundbehandlung nach Anspruch 1 oder 2, **dadurch gekennzeichnet, dass** es sich bei der Salbe um ein einphasiges lipophiles System oder um ein wasserhaltiges Hydrogel handelt.

4. Medizinisch-topische Zusammensetzung zur Wundbehandlung nach Anspruch 1 bis 3, **dadurch gekennzeichnet, dass** es sich bei den natürlichen Ölen um Leinsamenöl mit folgender Fettsäure-Zusammensetzung handelt:
Palmitinsäure 4-8 %,
Stearinsäure 2-5 %,
Ölsäure 16-26 %,
Linolsäure 14-24%,
Linolensäure 43-55 % sowie Fettsäuren mit Kohlenstoffketten, die mehr als 18 Kohlenstoffatome aufweisen 0,1-2 % und wobei sich die einzelnen Komponenten auf 100 % addieren.

5. Medizinisch-topische Zusammensetzung zur Wundbehandlung nach einem der Ansprüche 1-4, **dadurch gekennzeichnet, dass** der Anteil an Fettsäure-Extrakt aus den natürlichen Ölen 0,005 bis 1,5 Gew.-%, insbesondere 0,015 bis 0,3 Gew.-% bezogen auf das Gesamtgewicht der medizinisch-topischen Zusammensetzung beträgt.

6. Medizinisch-topische Zusammensetzung zur Wundbehandlung nach einem der Ansprüche 1-5, **dadurch gekennzeichnet, dass** es sich um ein einphasiges lipophiles System handelt, das mindestens einen Bestandteil ausgewählt aus der Gruppe umfassend Na-Carboxymethylcellulose, amorphes Siliciumdioxid, teilweise oder vollständig verestertes Glycerin, Polyethylenglykol, ethoxylierte höhere Alkohole, natürliche oder synthetische Wachse, Paraffin, Gemische aus höheren aliphatischen Alkanen, Ester aus Benzoesäure, Zimtsäure oder Benzylalkohol und Mischungen davon enthält.

7. Medizinisch-topische Zusammensetzung zur Wundbehandlung nach einem der Ansprüche 1-5, **dadurch gekennzeichnet, dass** es sich um wasserhaltiges Hydrogel handelt, wobei das wasserhaltige Hydrogel mindestens ein gelbildendes Polymer und mindestens einen mehrwertigen Alkohol umfasst.

8. Medizinisch-topische Zusammensetzung zur Wundbehandlung nach Anspruch 7, **dadurch gekennzeichnet, dass** das wasserhaltige Hydrogel mindestens ein gelbildendes Polymer, mindestens einen mehrwertigen Alkohol und mindestens ein Acrylsäurederivat umfasst, wobei das gelbildende Polymer ein Polysaccharid ist.

9. Medizinisch-topische Zusammensetzung zur Wundbehandlung nach Anspruch 7, **dadurch gekennzeichnet, dass** das wasserhaltige Hydrogel mindestens ein gelbildendes Polymer und mindestens einen mehrwertigen Alkohol umfasst, wobei das gelbildende Polymer eine Mischung aus einem Vorpolymer mit Isophorondiisocyanatendgruppen und einem Diamin auf Polyethylenoxidbasis ist und das Verhältnis der reaktiven Gruppen des Isocyanats zu den Amidgruppen des Diamins bei 1:1.25 bis 1:1.35 liegt.

10. Medizinisch-topische Zusammensetzung zur Wundbehandlung nach einem der Ansprüche 7-9, **dadurch gekennzeichnet, dass** das wasserhaltige Hydrogel als Lösungsvermittler ein Tensid enthält.

11. Vorrichtung zur Wundbehandlung, enthaltend eine medizinisch-topische Zusammensetzung nach einem der Ansprüche 1-10, **dadurch gekennzeichnet, dass** die medizinisch-topische Zusammensetzung auf eine Wundauflage aufgetragen vorliegt.

12. Vorrichtung zur Wundbehandlung nach Anspruch 11, wobei die Wundauflage ein oder mehrere Materialien ausgewählt aus Polyamid, Viskose und Baumwolle umfasst und sekretbindende Eigenschaften aufweist.

13. Vorrichtung zur Wundbehandlung nach Anspruch 11, wobei die Wundauflage ein oder mehrere Materialien ausgewählt aus Polyester und Polyurethan umfasst und sekretdurchlässige Eigenschaften aufweist.

14. Vorrichtung zur Wundbehandlung nach einem der Ansprüche 11-13, wobei die Wundauflage mit elementarem Silber und/oder mit Aktivkohle beschichtet ist.

15. Vorrichtung zur Wundbehandlung nach einem der Ansprüche 11-14, **dadurch gekennzeichnet, dass** die medizinisch-topische Zusammensetzung ein einphasiges lipophiles System ist, das gleichmäßig auf der Wundauflage verteilt ist, in einer Menge an 100-350 g einphasiges lipophiles System / m² Wundauflage, bevorzugt 125-300 g einphasiges lipophiles System / m² Wundauflage, weiter bevorzugt 150-270 g einphasiges lipophiles System / m² Wundauflage.

16. Medizinisch-topische Zusammensetzung zur Wundbehandlung nach einem der Ansprüche 1-10 oder Vorrichtung zur Wundbehandlung nach einem der Ansprüche 11-15 zur Verwendung zur beschleunigten Zellmigration während der Epithelisierungsphase.

## Claims

1. A medical-topical composition for wound treatment comprising 0.001 to 3% by weight of fatty acid extracts of natural oils based on the total weight of the medical-topical composition, **characterized in that** the natural oils are linseed oil or rapeseed oil, wherein medical-topical compositions containing a combination of active ingredients that consist of the following components
a) 0.01 to 20% by weight of almond oil,
b) 0.01 to 1.5% by weight of linseed oil fatty acids,
c) 0.001 to 0.5% by weight of amino acids; and
d) 0.01 to 1.5% by weight of creatine
are excluded.

2. The medical-topical composition for wound treatment according to claim 1, **characterized in that** the medical-topical composition is an ointment.

3. The medical-topical composition for wound treatment according to claim 1 or 2, **characterized in that** the ointment is a one-phase lipophilic system or a hydrous hydrogel.

4. The medical-topical composition for wound treatment according to claim 1 to 3, **characterized in that** the natural oils are linseed oil having the following fatty acid composition:
palmitic acid 4-8%,
stearic acid 2-5%,
oleic acid 16-26%,
linoleic acid 14-24%,
linolenic acid 43-55% as well as fatty acids with carbon chains having more than 18 carbon atoms 0.1-2% and wherein the individual components add up to 100%.

5. The medical-topical composition for wound treatment according to any of claims 1-4, **characterized in that** the proportion of fatty acid extract of the natural oils is 0.005 to 1.5% by weight, particularly 0.015 to 0.3% by weight based on the total weight of the medical-topical composition.

6. The medical-topical composition for wound treatment according to any of claims 1-5, **characterized in that** it is a single-phase lipophilic system containing at least one component selected from the group comprising Na carboxymethylcellulose, amorphous silica, partially or completely esterified glycerin, polyethylene glycol, ethoxylated higher alcohols, natural or synthetic waxes, paraffin, mixtures of higher aliphatic alkanes, esters of benzoic acid, cinnamic acid or benzyl alcohol, and mixtures thereof.

7. The medical-topical composition for wound treatment according to any of claims 1-5, **characterized in that** it is a hydrous hydrogel, wherein the hydrous hydrogel comprises at least one gel-forming polymer and at least one polyol.

8. The medical-topical composition for wound treatment according to claim 7, **characterized in that** the hydrous hydrogel comprises at least one gel-forming polymer, at least one polyol, and at least one acrylic acid derivative, wherein the gel-forming polymer is a polysaccharide.

9. The medical-topical composition for wound treatment according to claim 7, **characterized in that** the hydrous hydrogel comprises at least one gel-forming polymer and at least one polyol, wherein the gel-forming polymer is a mixture of a pre-polymer with isophorone diisocyanate end groups and a polyethylene oxide-based diamine and the ratio of reactive groups of the isocyanate to the amide groups of the diamine is 1:1.25 to 1:1.35.

10. The medical-topical composition for wound treatment according to any of claims 7-9, **characterized in that** the hydrous hydrogel contains a surfactant as solubilizer.

11. A device for wound treatment containing a medical-topical composition according to any of claims 1-10, **characterized in that** the medical-topical composition is present applied to a wound dressing.

12. The device for wound treatment according to claim 11, wherein the wound dressing comprises one or more materials selected from polyamide, viscose, and cotton and has secretion binding properties.

13. The device for wound treatment according to claim 11, wherein the wound dressing comprises one or more materials selected from polyester and polyurethane and has secretion permeable properties.

14. The device for wound treatment according to any of claims 11-13, wherein the wound dressing is coated with elementary silver and/or activated carbon.

15. The device for wound treatment according to any of claims 11-14, **characterized in that** the medical-topical composition is a single-phase lipophilic system that is evenly distributed on the wound dressing in an amount of 100-350 g of the single-phase lipophilic system/m² of the wound dressing, preferably 125-300 g of the single-phase lipophilic system/m² of the wound dressing, more preferably 150-270 g of the single-phase lipophilic system/m² of the wound dressing.

16. The medical-topical composition for wound treatment according to any of claims 1-10 or the device for wound treatment according to any of claims 11-15 for use in accelerated cell migration during the epithelialization phase.

## Revendications

1. Composition médicinale topique pour le traitement de plaies, comprenant 0,001 à 3 % en poids d'extraits d'acide gras d'huiles naturelles par rapport au poids total de la composition médicinale topique, **caractérisée en ce que** les huiles naturelles sont l'huile de lin ou l'huile de colza, dans laquelle sont exclues des compositions médicinales topiques qui contiennent une combinaison de principes actifs qui consistent en composants suivants
a) 0,01 à 20 % en poids d'huile d'amande,
b) 0,01 à 1,5 % en poids d'acides gras d'huile de lin,
c) 0,001 à 0,5 % en poids d'acides aminés et
d) 0,01 à 1,5 % en poids de créatine.

2. Composition médicinale topique pour le traitement de plaies selon la revendication 1, **caractérisée en ce que** la composition médicinale topique est une pommade.

3. Composition médicinale topique pour le traitement de plaies selon la revendication 1 ou 2, **caractérisée en ce que** la pommade est un système monophasique lipophile ou un hydrogel contenant de l'eau.

4. Composition médicinale topique pour le traitement de plaies selon une revendication 1 à 3, **caractérisée en ce que** les huiles naturelles sont une huile de lin ayant une composition d'acides gras suivante :
acide palmitique 4 à 8 %,
acide stéarique 2 à 5 %,
acide oléique 16 à 26 %
acide linoléique 14 à 24 %,
acide linolénique 43 à 55 % ainsi que des acides gras comportant des chaînes de carbone qui présentent plus de 18 atomes de carbone à 0,1 à 2 % et dans laquelle les composants individuels s'additionnent pour donner 100 %.

5. Composition médicinale topique pour le traitement de plaies selon l'une des revendications 1 à 4, **caractérisée en ce que** la proportion d'extrait d'acide gras des huiles naturelles est de 0,005 à 1,5 % en poids, en particulier 0,015 à 0,3 % en poids par rapport au poids total de la composition médicinale topique.

6. Composition médicinale topique pour le traitement de plaies selon l'une des revendications 1 à 5, **caractérisée en ce qu'**il s'agit d'un système monophasique lipophile qui contient au moins un composant choisi dans le groupe comprenant de la carboxyméthylcellulose de sodium, du dioxyde de silicium amorphe, de la glycérine partiellement ou complètement estérifiée, du polyéthylène glycol, des alcools supérieurs éthoxylés, des cires naturelles ou synthétiques, de la paraffine, des mélanges d'alcanes aliphatiques supérieurs, des esters d'acide benzoïque, de l'acide cinnamique ou de l'alcool benzylique et des mélanges de ceux-ci.

7. Composition médicinale topique pour le traitement de plaies selon l'une des revendications 1 à 5, **caractérisée en ce qu'**il s'agit d'un hydrogel contenant de l'eau, dans lequel l'hydrogel contenant de l'eau comprend au moins un polymère formant un gel et au moins un alcool multivalent.

8. Composition médicinale topique pour le traitement de plaies selon la revendication 7, **caractérisée en ce que** l'hydrogel contenant de l'eau comprend au moins un polymère formant un gel, au moins un alcool multivalent et au moins un dérivé d'acide acrylique, dans laquelle le polymère formant un gel est un polysaccharide.

9. Composition médicinale topique pour le traitement de plaies selon la revendication 7, **caractérisée en ce que** l'hydrogel contenant de l'eau comprend au moins un polymère formant un gel et au moins un alcool multivalent, dans laquelle le polymère formant un gel est un mélange d'un pré-polymère comportant des groupes diisocyanate d'isophorone et une diamine à base d'oxyde de polyéthylène et le rapport des groupes réactifs de l'isocyanate aux groupes amide de la diamine est de 1:1,25 à 1:1,35.

10. Composition médicinale topique pour le traitement de plaies selon l'une des revendications 7 à 9, **caractérisée en ce que** l'hydrogel contenant de l'eau contient un tensioactif servant d'agent de solubilisation.

11. Dispositif pour le traitement de plaies, contenant une composition médicinale topique selon l'une des revendications 1 à 10, **caractérisé en ce que** la composition médicinale topique est appliquée sur un pansement.

12. Dispositif pour le traitement de plaies selon la revendication 11, dans lequel le pansement comprend un ou plusieurs matériaux choisis parmi le polyamide, la viscose et le coton et présente des propriétés de liaison aux sécrétions.

13. Dispositif pour le traitement de plaies selon la revendication 11, dans lequel le pansement comprend un ou plusieurs matériaux choisis parmi le polyester et le polyuréthane et présente des propriétés de liaison aux sécrétions.

14. Dispositif pour le traitement de plaies selon l'une des revendications 11 à 13, dans lequel le pansement est revêtu d'argent élémentaire et/ou de charbon actif.

15. Dispositif pour le traitement de plaies selon l'une des revendications 11 à 14, **caractérisé en ce que** la composition médicinale topique est un système monophasique lipophile qui est réparti uniformément sur le pansement, en une quantité de 100 à 350 g de système monophasique lipophile / m² de pansement, de préférence de 125 à 300 g de système monophasique lipophile / m² de pansement, de manière davantage préférée, de 150 à 270 g de système monophasique lipophile / m² de pansement.

16. Composition médicinale topique pour le traitement de plaies selon l'une des revendications 1 à 10 ou dispositif pour le traitement de plaies selon l'une des revendications 11 à 15 pour son utilisation pour accélérer la migration cellulaire pendant la phase d'épithélialisation.
